# EUROPEAN PATENT APPLICATION

(11) **EP 4 276 188 A1**
(43) Date of publication of application: **15.11.2023**
(21) Application number: 21924111.4
(22) Date of filing: 06.07.2021
(51) Int. Cl.: C12P 13/00, C12P 17/12, C12P 17/14, C12N 9/10

(54) **METHOD FOR SYNTHESIZING CHIRAL AMINE COMPOUND**

(30) Priority: 04.02.2021 CN 202110151062; 26.04.2021 CN 202110451383
(71) Applicant: Asymchem Laboratories (Tianjin) Co., Ltd., Tianjin 300457 (CN)
(72) Inventor: HONG, Hao, Morrisville North Carolina 27560 (US); JAMES, Gage, Morrisville, North Carolina 27560 (US); XIAO, Yi, Tianjin 300457 (CN); ZHANG, Na, Tianjin 300457 (CN); JIAO, Xuecheng, Tianjin 300457 (CN); MA, Yulei, Tianjin 300457 (CN); MOU, Huiyan, Tianjin 300457 (CN); WANG, Zujian, Tianjin 300457 (CN); SUN, Kaihua, Tianjin 300457 (CN); JIA, Ru, Tianjin 300457 (CN); LIU, Fang, Tianjin 300457 (CN); LIU, Wenjing, Tianjin 300457 (CN)
(74) Representative: Studio Torta S.p.A.
(86) International application number: PCT/CN2021/104842
(87) International publication number: WO 2022/166104

(57) **Abstract**

Provided is a method for synthesizing a chiral amine compound. A transaminase is used to transaminate a ketone substrate under the action of an amino donor, to obtain the chiral amine compound; and the conserved amino acid sequence region of the transaminase at least includes a region 1 (MAGLWCVN) and a region 2 (YNTFFKT). With the transaminase with the specific conserved amino acid sequence region to synthesize a large sterically hindered chiral amine, the enzyme catalytic reaction volume is small, the synthesizing route is short, the product yield is high, a high-cost noble metal is not required for catalysis under the synthesizing conditions, three wastes are reduced, and the production cost is saved.

## Description

### Technical Field

The present disclosure relates to the field of chiral amine synthesis, in particular to a method for synthesizing a chiral amine compound.

### Background

Large sterically hindered chiral amine compounds (in the present application, it refers to a class of compounds with a group next to a latent chiral carbonyl that is greater than a methyl) are a class of important chiral intermediates widely used in the synthesis of optically active substances, functional molecules, drugs, and ligands. However, there are currently few reports on the synthesizing of such chiral compounds, and satisfactory results may not be achieved. For example, the selectivity of phenyl tert-butyl amino ester with a large steric hindrance on one side of the carbonyl is 76% (Angelw. Chem. Int. Ed. 2007, 46, 4367). At present, some metal catalysts, such as ruthenium, rhodium, and palladium, all have the better stereoselectivity reported for an asymmetric hydrogenation reaction with a small steric hindrance (in the present application, it refers to a class of compounds with a group next to the latent chiral carbonyl that is H or methyl). However, as the approached steric hindrance of the carbonyl becomes larger, the catalytic effect becomes worse.

The asymmetric hydrogenation is a type of technologies for production of fine chemicals and drug precursors. However, this technology has some drawbacks, such as the need to use a highpressure hydrogen gas, it poses a great challenge to production safety and requires expensive transition metal catalysts. These toxic catalysts also need to be carefully treated and removed in the post-treatment process. In addition, this process requires a lot of human and material resources to screen corresponding ligands. In many cases, there is still a problem of low stereoselectivity in this process (Science, 2010, 329, 305). Some reactions also require protection and deprotection of active groups.

It may be seen that synthesizing methods in prior art have the high costs and require the protection and deprotection of the active groups, the reaction conditions are harsh, and noble metal catalysts are used. It is necessary to provide a new synthesizing method.

### Summary

A main purpose of the present disclosure is to provide a method for synthesizing a chiral amine compound, as to solve problems in prior art that the method for synthesizing the chiral amine compound is high in cost and harsh in reaction condition.

In order to achieve the above purpose, according to one aspect of the present disclosure, a method for synthesizing a chiral amine compound is provided, and this synthesizing method uses a transaminase to perform a transamination action on a ketone substrate shown in Formula I under the action of an amino donor, to obtain the chiral amine compound:

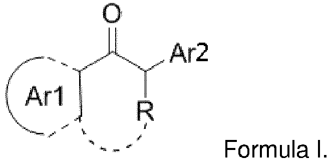

Ar1 represents a substituted or unsubstituted aryl, a substituted or unsubstituted arylene, or a substituted or unsubstituted heteroarylene; Ar2 represents a substituted or unsubstituted aryl, a substituted or unsubstituted cycloalkyl, or a substituted or unsubstituted chain alkyl or heteroalkyl; and R is optional, herein R represents a substituted or unsubstituted alkylene with a C atomic number of 1∼5, and R is connected with Ar1 to form a ring; herein, substituents in the substituted aryl, substituted arylene, substituted heteroarylene, or substituted alkylene are each independently selected from a halogen, a hydroxyl, an amino group, or -S-CH₃, and the heteroatoms in the heteroarylene and heteroalkyl are each independently selected from N, O, or S; herein, the transaminase is a type of transaminases with a conserved amino acid sequence region, the conserved amino acid sequence region at least includes a region 1 and a region 2, the conserved amino acid sequence of the region 1 is MAGLWCVN, and the conserved amino acid sequence of the region 2 is YNTFFKT; or the transaminase is *Chromobaterium violaceum* shown in SEQ ID NO: 1 and a mutated transaminase of any one transaminases shown in SEQ ID NO: 2 to SEQ ID NO: 55 of which W and/or C in the conserved amino acid sequence of the region 1 is mutated into A; or the transaminase is a transaminase which has the same source as any one of the transaminases shown in SEQ ID NO: 1 to SEQ ID NO: 55 and has 75%, preferably 85%, more preferably more than 95% of identity on the premise of having the region 1 and the region 2.

Further, the transaminase is any one of the transaminases shown in SEQ ID NO: 1 to SEQ ID NO: 55.

Further, the substituent in the substituted aryl or arylene is each individually selected from a halogen or -S-CH₃, the halogen or -S-CH₃ is located at any one or more positions in an orthoposition, a meta-position, or a para-position of the aryl or arylene, and preferably, the halogen is F, Cl, or Br.

Further, Ar1 represents a substituted or unsubstituted aryl or arylene, Ar2 represents an unsubstituted aryl, R represents an unsubstituted alkylene with a C atomic number of 1∼5, and the R is connected with Ar1 to form a ring.

Further, Ar1 represents an unsubstituted heteroarylene, Ar2 represents a halogen-substituted aryl, R represents a hydroxyl-substituted alkylene with a C atomic number of 1∼5, and the R is connected with Ar1 to form a ring.

Further, Ar1 represents an arylene substituted by halogen and amino group, and Ar2 represents a cycloalkyl with a C atomic number of 3~8.

Further, Ar1 represents an unsubstituted cycloalkyl or aryl, and Ar2 represents a substituted or unsubstituted cycloalkyl or aryl.

Further, Ar1 represents a hydroxyl, a methyl, an ethyl, or a -S-CH₃-substituted cycloalkyl or aryl, and Ar2 represents an unsubstituted aryl or alkyl.

Further, the ketone substrate is:

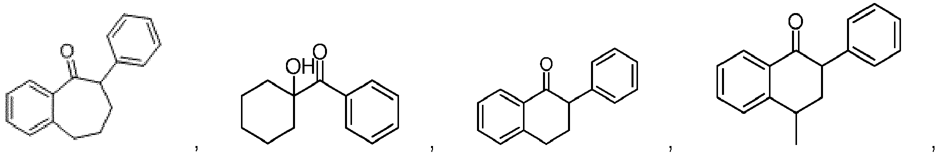

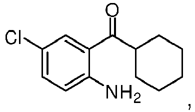

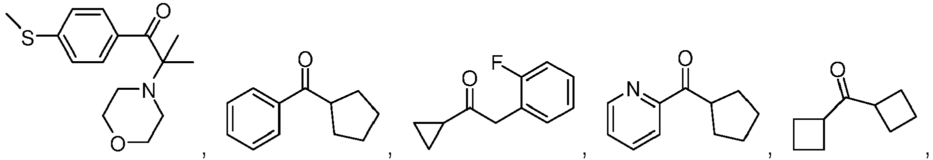

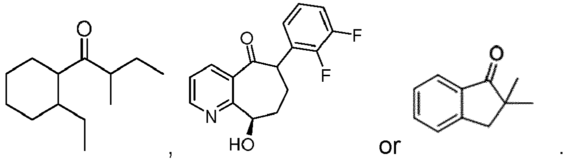

Further, the amino donor is isopropylamine, isopropylamine hydrochloride, alanine, n-butylamine, or aniline.

By applying technical schemes of the present disclosure, the large sterically hindered chiral amine is synthesized with a type of the transaminases having the specific conserved amino acid sequence regions. The enzyme catalytic reaction volume is small, the synthesizing route is short, the product yield is high, and a high-cost noble metal is not required for catalysis under the synthesizing conditions. In addition, three wastes are greatly reduced, and the production cost is saved.

### Brief Description of the Drawings

Drawings of the description for constituting a part of the present application are used to provide further understanding of the present disclosure. Schematic embodiments of the present disclosure and descriptions thereof are used to explain the present disclosure, and do not constitute improper limitation to the present disclosure. In the drawings:
Fig. 1 shows a schematic diagram of partial results of multi-sequence (SEQ ID NO: 1 and SEQ ID NO: 2 to SEQ ID NO: 19) alignment according to an embodiment of the present disclosure.
Fig. 2 shows a schematic diagram of partial results of multi-sequence (SEQ ID NO: 20 to SEQ ID NO: 37) alignment according to an embodiment of the present disclosure.
Fig. 3 shows a schematic diagram of partial results of multi-sequence (SEQ ID NO: 38 to SEQ ID NO: 55) alignment according to an embodiment of the present disclosure.

### Detailed Description of the Embodiments

It should be noted that in the case without conflicting, embodiments in the present application and features in the embodiments may be combined with each other. The present disclosure is described in detail below in combination with the embodiments.

In order to solve problems in prior art that the synthesizing method for the large sterically hindered chiral amine is high in cost and harsh in reaction condition, the inventor of the present application tries to use a method of a biological enzyme to synthesize, but a transaminase naturally has two pockets in structure, so a substrate which may be converted by it is relatively limited. No transaminase with catalytic activity for large sterically hindered chiral amine compounds has been reported yet in the prior art. In order to improve this situation, the inventor conducted a screening on the existing transaminases reported already in large quantities, and screens thousands of the wild-type transaminases, most of which do not have the catalytic activity (namely a target product may not be detected at all), but some transaminases that may catalyze the synthesizing of such large sterically hindered chiral amine compounds are still found. Although the activity is low, a basis for potential improvement is at least provided.

These transaminases are a CvTA wild-type transaminase (SEQ ID NO: 1) of *Chromobaterium violaceum*, a transaminase which is the same genus but different species from the CvTA wild-type transaminase and a transaminase which is the different genus and the different species from the CvTA wild-type transaminase. Whether it is the transaminase (as shown in Table 1) which is the same genus but different species from the CvTA wild-type transaminase, or the transaminase (as shown in Table 2) which is the different genus and the different species from the CvTA wild-type transaminase, these wild-type transaminases all have certain activity of catalyzing the conversion of the large sterically hindered substrates into the chiral amine compounds, although the activity is low (less than 1%).

The sequence of SEQ ID NO: 1 is as follows (459 amino acids):

MQKQRTTSQWRELDAAHHLHPFTDTASLNQAGARVMTRGEGVYLWDSEGNKIIDGMAGL
WCVNVGYGRKDFAEAARRQMEELPFYNTFFKTTHPAVVELSSLLAEVTPAGFDRVFYTNSGSE
SVDTMIRMVRRYWDVQGKPEKKTLIGRWNGYHGSTIGGASLGGMKYMHEQGDLPIPGMAHIE
QPWWYKHGKDMTPDEFGVVAARWLEEKILEIGADKVAAFVGEPIQGAGGVIVPPATYWPEIERI
CRKYDVLLVADEVICGFGRTGEWFGHQHFGFQPDLFTAAKGLSSGYLPIGAVFVGKRVAEGLIA
GGDFNHGFTYSGHPVCAAVAHANVAALRDEGIVQRVKDDIGPYMQKRWRETFSRFEHVDDVR
GVGMVQAFTLVKNKAKRELFPDFGEIGTLCRDIFFRNNLIMRACGDHIVSAPPLVMTRAEVDEM
LAVAERCLEEFEQTLKARGLA.

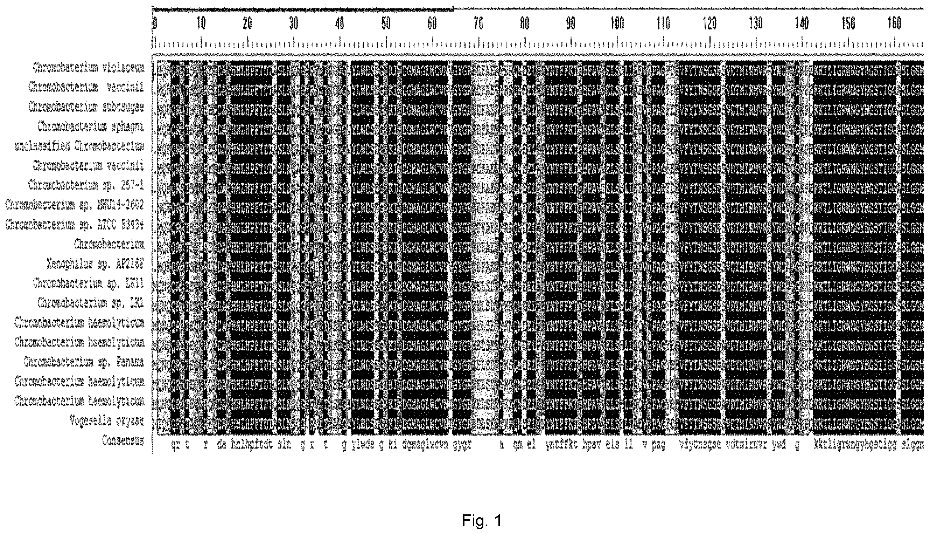

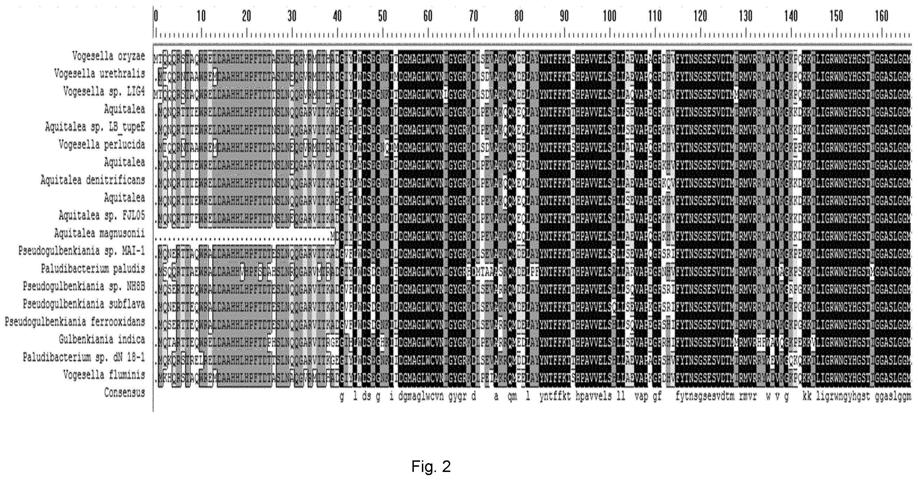

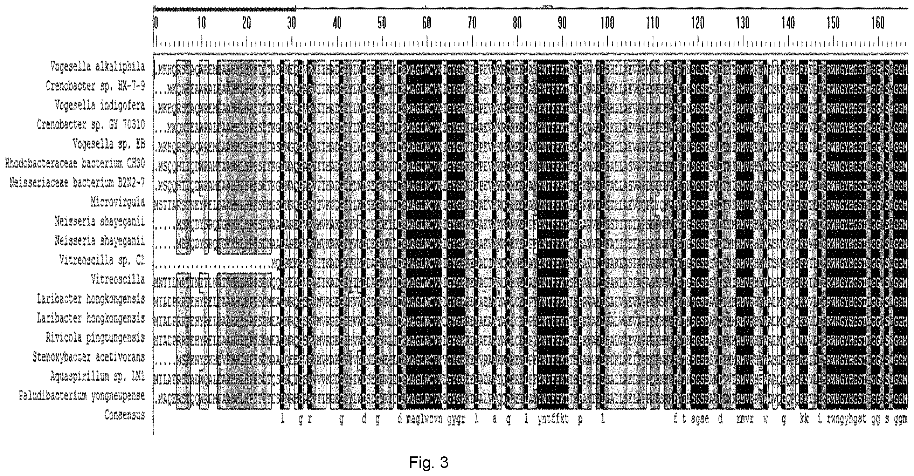

The inventor further performs the sequence analysis on these transaminases with the activity, and it was found that there is a plurality of highly conserved amino acid regions in these transaminases, as shown in Fig. 1, Fig. 2 and Fig. 3. The sequence identities of these conserved regions reach more than 95%, some reach more than 98%, and some even reach 100%.

Further, the inventor finds from experiments that these conserved amino acid regions are the commonness of the catalytic activity of these transaminases for the catalytic synthesizing of the large sterically hindered chiral amines. In order to further verify this hypothesis, the inventor selected one or more amino acids from the above common conserved amino acid regions and replaced it. It was found that after these conserved amino acid regions are mutated, a transaminase mutant with improved catalytic activity may be obtained. So far, the inventor proved to discover a type of the transaminases that may catalyze the synthesizing of the large sterically hindered chiral amines.

Based on the above research results, the applicant proposes a technical scheme of the present application. In a preferred embodiment of the present application, a method for synthesizing a chiral amine compound is provided, and the above modified transaminase mutant of the present application is used to perform a transamination action on a ketone substrate shown in Formula I under the action of an amino donor, to obtain the chiral amine compound; 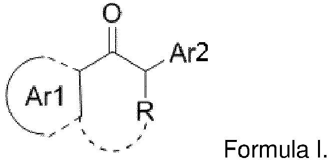
Ar1 represents a substituted or unsubstituted aryl, a substituted or unsubstituted arylene, or a substituted or unsubstituted heteroarylene;
Ar2 represents a substituted or unsubstituted aryl, a substituted or unsubstituted cycloalkyl, or a substituted or unsubstituted chain alkyl or heteroalkyl; and
R is optional, herein R represents a substituted or unsubstituted alkylene with a C atomic number of 1~5, and R is connected with Ar1 to form a ring;
herein, substituents in the substituted aryl, substituted arylene, substituted heteroarylene, or substituted alkylene are each independently selected from a halogen, a hydroxyl, an amino group, and -S-CH₃, and heteroatoms in the heteroarylene and heteroalkyl are each independently selected from N, O, or S; herein, the transaminase is a type of transaminases with conserved amino acid sequence region, the conserved amino acid sequence region at least includes a region 1 and a region 2, the conserved amino acid sequence of the region 1 is: MAGLWCVN, and the conserved amino acid sequence of the region 2 is: YNTFFKT.

As described above, such transaminases with certain catalytic activity to the large sterically hindered substrate have a plurality of conserved amino acid regions, and two conserved amino acid sequence regions defined above have 100% of the identity among all detected subsequent transaminases with certain catalytic activity. In addition, it was provided from preliminary tests that such conserved amino acid sequence regions are a candidate region for promoting the activity of such substrates. Therefore, the transaminase with the above conserved amino acid sequence regions has the catalytic activity for catalyzing the large sterically hindered substrates, and is expected to be transformed into a transaminase with higher activity, the synthesizing efficiency of such large sterically hindered chiral amine compounds is further improved, the production cost is reduced and three wastes are reduced.

It may be seen from Figs. 1 to 3 that in addition to the above two conserved amino acid sequence regions, there are other conserved amino acid sequence regions of this type of the transaminase. Therefore, in a preferred embodiment, the above transaminase further includes a region 3, and the conserved amino acid sequence of the region 3 is: RWNGYHGST.

In a preferred embodiment, the above transaminase is *Chromobaterium violaceum* shown in SEQ ID NO: 1 and a mutated transaminase of any one transaminase shown in SEQ ID NO: 2 to SEQ ID NO: 55 in the table below of which W and/or C in the conserved amino acid sequence of the region 1 is mutated into A; or the above transaminase is a transaminase which has 75%, preferably 85%, more preferably more than 95% of the identity with the transaminase shown in SEQ ID NO: 1 derived from *Chromobaterium violaceum* and any one of the transaminases shown in SEQ ID NO: 2 to SEQ ID NO: 55 in Table 1 and Table 2, and has the region 1 and the region 2.

In a more preferred embodiment, the above transaminase is a transaminase shown in SEQ ID NO: 1 derived from *Chromobaterium violaceum* and any one of transaminases shown in SEQ ID NO: 2 to SEQ ID NO: 55 in Table 1 and Table 2.

The large sterically hindered substrate mentioned in the present application refers to a class of compounds with a group next to a latent chiral carbonyl that is larger than a methyl, and any compounds conforming to the above definition may be used as the substrates catalyzed by such transaminases. In a preferred embodiment, the substituent in the substituted aryl or arylene is a halogen or -S-CH₃, the halogen or -S-CH₃ is located at any one or more positions in an ortho-position, a meta-position, or a para-position of the aryl or arylene, and preferably, the halogen is F, Cl, or Br.

In a preferred embodiment, Ar1 represents a substituted or unsubstituted aryl or arylene, Ar2 represents an unsubstituted aryl, R represents an unsubstituted alkylene with a C atomic number of 1~5, and the R is connected with Ar1 to form a ring.

In a preferred embodiment, Ar1 represents an unsubstituted heteroarylene, Ar2 represents a halogen-substituted aryl, R represents a hydroxyl-substituted alkylene with a C atomic number of 1~5, and R is connected with Ar1 to form a ring.

In a preferred embodiment, Ar1 represents an arylene substituted by halogen and amino group, and Ar2 represents a cycloalkyl with a C atomic number of 3~8.

In a preferred embodiment, Ar1 represents an unsubstituted cycloalkyl or aryl, and Ar2 represents a substituted or unsubstituted cycloalkyl or aryl.

In a preferred embodiment, Ar1 represents a hydroxyl, a methyl, an ethyl, or a -S-CH₃-substituted cycloalkyl or aryl, and Ar2 represents an unsubstituted aryl or alkyl.

In a preferred embodiment, the ketone substrate is:

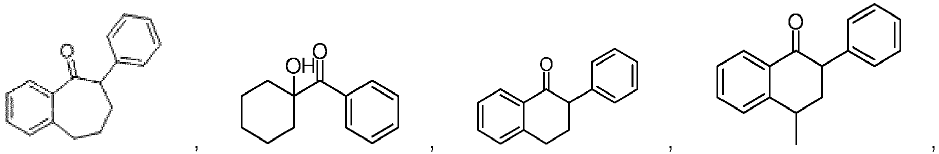

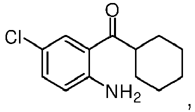

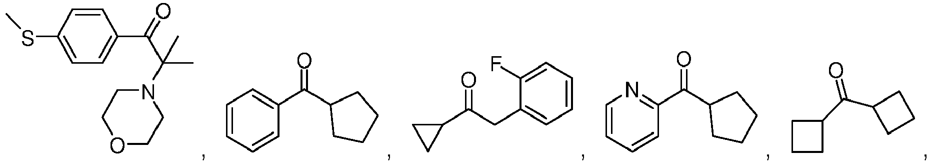

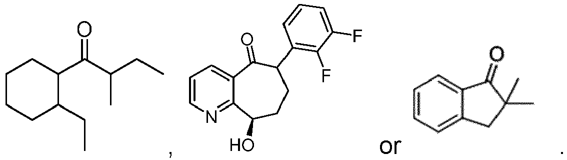

In a preferred embodiment, the amino donor is isopropylamine, isopropylamine hydrochloride, alanine, n-butylamine, or aniline.

The above technical schemes and technical effects are described below in combination with specific embodiments.

Substrate 1: 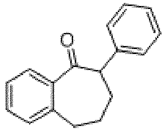
(I) Part one: the catalytic activities of wild-type transaminases from different species and genera to such large sterically hindered substrates are screened and verified.

### Embodiment 1: Screening of wild-type transaminases

In this embodiment, the transaminase derived from *Chromobaterium violaceum* (the sequence was shown in SEQ ID NO: 1, marked as the CvTA wild-type transaminase) was firstly selected, and its catalytic activity to synthesize the target large sterically hindered substrate (Substrate 1) was tested. Herein, the detailed process of catalyzing Substrate 1 by the CvTA wild-type transaminase was as follows: 1 mL of a system included 1 mg of Substrate 1, 0.1 mg of PLP, 1 mg of isopropylamine hydrochloride, 50 mg of enzyme powder, 100 mM of pH8.0 phosphate buffer solution, and it was reacted at 30°C for 40 h.

Results showed that the catalytic efficiency of the CvTA wild-type transaminase for the synthesizing of Substrate 1 was 0.56%.

Secondly, the wild-type transaminase (specifically shown in Table 1) which was the same genus but different species from the above CvTA wild-type transaminase, and of which the amino acid sequence had more than 82% of the identity compared with SEQ ID NO: 1, was selected, and its catalytic activity was tested. The catalytic process was the same as above. It was found from results that it was similar to the CvTA wild-type transaminase, the catalytic efficiency of these wild-type transaminase strains for the synthesizing of the target large sterically hindered substrate (Substrate 1) was 0.01%∼1%. Specific results were shown in Table 1.

**Table 1: Other transaminases derived from Chromobacterium and identity thereof with sequence of Chromobacterium violaceum.**

| No. | Source | NCBI Seq ID | SEQ ID NO: | Identity(%) | Conversion rate |
|---|---|---|---|---|---|
| 1 | Chromnbacterium vaccinii | WP_046156378.1 | 2 | 95.21 | +++ |
| 2 | Chromobacterium subtsugae | WP_047243123.1 | 3 | 93.67 | + |
| 3 | Chromobacterium sphagni | WP_011116856.1 | 4 | 92.79 | ++ |
| 4 | unclassified Chromobacterium | WP_114062556.1 | 3 | 95.86 | + |
| 5 | Chromobacterium vaccinii | WP_104946997.1 | 6 | 95.21 | ++ |
| 6 | Chromobacterium sp. 257-1 | WP_149795777.1 | 7 | 94.76 | + |
| 7 | Chromobacterium sp. MWU14-2602 | WP_103903523.1 | 9 | 94.76 | ++ |
| 8 | Chromabacterium sp. ATCC 53434 | WP_101708025.1 | 9 | 94.54 | ++ |
| 9 | Chromobacterium | WP_043629242.1 | 10 | 94.1 | + |
| 10^{∗} | Xencphilus sp. AP218F | WP_088737038.1 | 11 | 87.58 | ++ |
| 11 | Chromobacterium sp. LK11 | WP_049412320.1 | 12 | 84.25 | ++ |
| 12 | Chromobacterium sp. LK1 | WP_048411976.1 | 13 | 83.19 | ++ |
| 13 | Chromobacterium haemlyticum | WP_081556739.1 | 14 | 82.97 | + |
| 14 | Chromobacterium haemolyticum | OQ533371.1 | 15 | 83.15 | + |
| 15 | Chromobacterium sp. Panama | WP_10779474.1 | 16 | 82.75 | ++ |
| 16 | Chromobacterium haemolyticum | OQS37730.1 | 17 | 82.93 | ++ |
| 17 | Chromobacterium haemolyticum | WP_166453011.1 | 18 | 82.53 | ++ |

| | | | | | |
|---|---|---|---|---|---|
| 10*: the enzyme with the number 10 does not belong to the *Chromobacterium* genus, but it was listed in this table due to the high identity. | | | | | |

In the above table, + represented the conversion rate was 0.01%∼0.1%, ++ represented the conversion rate was 0.1-0.5%, and +++ represented the conversion rate was 0.5∼1%.

### Embodiment 2

In addition to the transaminase derived from *Chromobacterium*, the present application further tested transaminases from other sources having 69% to 87% of the identity with *Chromobaterium violaceum.* Specific information was listed in Table 2.

In this embodiment, the amino acid sequences of the wild-type transaminases from different sources between the 54-th site to the 63-th site and the 84-th site to the 96-th site also showed the high conserved property. These wild-type transaminases also had 0.01%∼1% of the catalytic activity for the target large sterically hindered substrate (Substrate 1) according to test results (shown in Table 2).

**Table 2: Other transaminases having lower identity with Chromobaterium violaceum, but being derived from different genera.**

| No. | Source | NCBI Seq ID | SEQ ID NO: | identity(%) | CR |
|---|---|---|---|---|---|
| 18 | Vogesellaoryzae | WP_174874069.1 | 19 | 83.19 | ++ |
| 19 | Vogesellawethralis | WP_144371715.1 | 10 | 82.14 | + |
| 20 | Vogesellasp LIG4 | WP_0SS967522.1 | 11 | 82.31 | ++ |
| 21 | Aquitalea | WP_089085350.1 | 22 | 81.7 | ++ |
| 22 | Aquitaleasp LB_tupeE | WP_178973970.1 | 23 | 81.7 | +++ |
| 23 | Vogesellaperlucida | WP_147687830.1 | 24 | 81.26 | + |
| 24 | Aquitalea | WP_03523625.1 | 25 | 81.48 | + |
| 25 | Aquitaleadenitrificans | WP_59877958.1 | 26 | 81.05 | + |
| 26 | Aquitalea | WP_045848621.1 | 27 | 81.05 | ++ |
| 27 | Aquitaleasp.FJL05 | WP_114643397.1 | 28 | 81.05 | ++ |
| 28 | Aguitaleamagnusonii | WP_0592873191 | 29 | 81.05 | ++ |
| 29 | Pseudogulbenkianiasp.MAI-1 | WP_024301818.1 | 30 | 80.35 | ++ |
| 30 | Paludibacteriunspaludis | WP_189532963.1 | 31 | 79.26 | + |
| 31 | Pseudogulbenkianiasp.NH8B | WP_014087389.1 | 12 | 79.04 | + |
| 32 | Pseudogulbenkianiasubflava | WP_385275708.1 | 33 | 79.48 | ++ |
| 33 | Pseudogulbenkianiaferrooxidans | WP_008952788.1 | 34 | 79.29 | +++ |
| 34 | Gulbenkianiaindica | WP_055434103.1 | 35 | 79.74 | ++ |
| 35 | Paludibacteriumsp.dN18-1 | MTD33855.1 | 34 | 7996 | +++ |
| 36 | Vogesellafluminis | WP_189352298.1 | 37 | 79.3 | ++ |
| 37 | Wogesllaalkaliphila | WP_189374996.1 | 38 | 78.87 | + |
| 38 | Crenabactersp.HX-7-9 | WP_163315775.1 | 39 | 79.04 | + |
| 39 | Vogesellaindigofera | WT_120809711.1 | 40 | 78.43 | ++ |
| 40 | Crenobactersp.GY70310 | WP_136552442.1 | 41 | 78.21 | + |
| 41 | Vogesellasp.EB | WP_047966302.1 | 42 | 77.38 | + |
| 42 | RbodobacterceaebacteriumCH30 | RQW28969.1 | 43 | 77.78 | ++ |
| 43 | NeisseriaceaebacteriumB2N2-7 | MXR37125.1 | 44 | 79.21 | ++ |
| 44 | Microvirgula | WP_028498438.1 | 45 | 79.21 | ++ |
| 4; | Neisseriashayeganii | WP_182122880.1 | 46 | 75.38 | ++ |
| 46 | Neisseriashayeganii | WP_00918141.1 | 47 | 70.24 | + |
| 47 | Vineoscillaps C1 | AAD41041.1 | 48 | 69.8 | +++ |
| 48 | Vitreoscilla | WP_019957606.1 | 49 | 70.2 | ++ |
| 49 | Laribacterhongkongensis | WP_041825665.1. | 50 | 71.21 | + |
| 50 | Laribacterhongkongensis | WP_19358443.5.1 | 51 | 71.21 | + |
| 51 | Rivicolapinghungensis | WP_110389486.1 | 52 | 70.99 | +- |
| 52 | Stenoxybacteracetivorans | WP_037587322.1 | 53 | 69.87 | ++ |
| 53 | Aquaspirilhunsp LM1 | WP_077397579.1 | 53 | 69.51 | ++ |
| 54 | Paludibacteriumyongneupense | WP_028535161.1 | 55 | 69.23 | ++ |

| | | | | | |
|---|---|---|---|---|---|
| CR: represent Conversion rate. | | | | | |

In the above table, + represented the conversion rate was 0.01%∼0.1%, ++ represented the conversion rate was 0.1∼0.5%, and +++ represented the conversion rate was 0.5∼1%.

Further, sequence alignment was performed on the different transaminase strains shown in Table 1 and Table 2, and alignment results were shown in Figs. 1 to 3. It might be seen from the results of multi-sequence alignment that the sequence of wild-type *Chromobaterium violaceum* transaminase (namely SEQ ID NO: 1) was used as a reference, two regions of the conserved amino acid sequence region from the 56-th site to the 63-th site and the conserved amino acid sequence region from the 84-th site to the 96-th site were highly conserved in the transaminases of listed species and genera, and were structurally located near the substrate binding site, which were relatively important amino acids. Therefore, it was speculated that these two conserved amino acid regions may improve the affinity of the large sterically hindered substrate, and it was helpful to further enhance the catalytic activity.

### Embodiment 3

In order to further confirm the influence of such transaminases with the above conserved amino acid regions on the catalytic activity of the large sterically hindered substrate, the inventor preliminarily performed a mutation A on the amino acids W and C in the conserved amino acid region 1 (the specific sequence was DGMAGLWCVNVGYGR) of the transaminase (namely SEQ ID NO: 1) derived from *Chromobaterium violaceum,* and found that the catalytic efficiency for Substrate 1 was improved after the mutation of these conserved amino acids, the conversion rate after the W-A mutation was increased to 5-10%, and the conversion rate after the C-A mutation was increased to 1∼5% (the specific reaction process was the same as recorded in Embodiment 1).

In order to identify the transaminase from other species sources, the conversion rate of the substrate was improved after the same mutation occurring at this conserved site. The inventor also performed the above same mutation on the transaminase from other sources, and detection results were shown in the table below.

**Table 3:**

| Enzyme No. | Mutant activity (W60A) | Mutant activity (C61A) |
|---|---|---|
| 1 | +++++ | ++++ |
| 2 | +++++ | ++++ |
| 3 | ++++ | +++++ |
| 4 | ++++ | ++++ |
| 5 | +++++ | ++++ |
| 6 | ++++ | ++++ |
| 7 | ++++ | ++++ |
| 8 | +++++ | ++++ |
| 9 | ++++ | +++++ |
| 10 | ++++ | ++++ |
| 11 | +++++ | ++++ |
| 12 | ++++ | ++++ |
| 13 | +++++ | ++++ |
| 14 | ++++ | ++++ |
| 15 | +++++ | ++++ |
| 16 | ++++ | +++++ |
| 17 | +++++ | ++++ |
| 18 | ++++ | ++++ |
| 19 | ++++ | ++++ |
| 20 | ++++ | +++++ |
| 21 | ++++ | ++++ |
| 22 | +++++ | ++++ |
| 23 | ++++ | +++++ |
| 24 | ++++ | ++++ |
| 25 | +++++ | ++++ |
| 26 | ++++ | +++++ |

In the above table, ++++ represented the conversion rate was 1%~5%, and +++++ represented the conversion rate was 5-10%.

From this, it might be seen that by further identifying the conserved amino acid sites on the conserved amino acid sequence regions and using it as a candidate feature sequence to enhance the catalytic activity, this feature sequence might be introduced into all wild-type strains. The above data showed that after this feature sequence was introduced, all wild-type transaminases had significantly enhanced catalytic activity on the target large sterically hindered substrate (Substrate 1).

### Embodiment 4

The inventor further detected the catalytic activity of different large sterically hindered substrates (it was specifically shown below from Substrate 2 to Substrate 13), 55 wild-type transaminases of the present disclosure and corresponding mutants after W-A and C-A mutations were used, and the catalytic activities of the following different substrates were detected under the reaction conditions similar to Embodiment 1. Substrate 2: 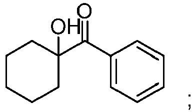 Substrate 3: 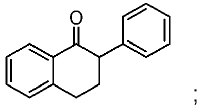 Substrate 4: 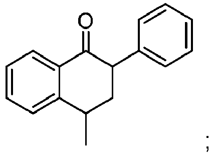 Substrate 5: 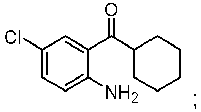 Substrate 6: 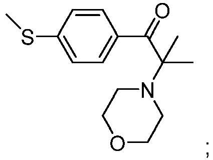 Substrate 7: 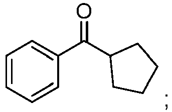 Substrate 8: 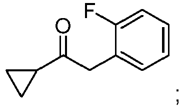 Substrate 9: 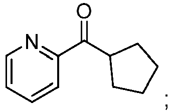 Substrate 10: 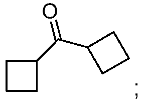 Substrate 11: 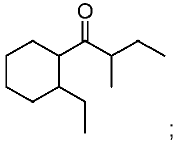 Substrate 12: 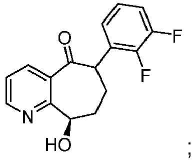 Substrate 13:

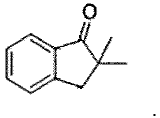

**Table 4: Catalysis of Substrate 2**

| Enzyme No. | Wild-type activity | Mutant activity (W60A) | Mutant activity (C61A) |
|---|---|---|---|
| CvTA | ++ | +++++ | ++++ |
| 1 | ++ | ++++ | +++++ |
| 2 | +++ | +++++ | +++++ |
| 3 | + | ++++ | ++++ |
| 4 | +++ | ++++ | ++++ |
| 5 | +++ | ++++ | ++++ |
| 6 | ++ | +++++ | ++++ |
| 7 | + | +++++ | ++++ |
| 8 | +++ | +++++ | ++++ |
| 9 | + | ++++ | +++++ |
| 10 | + | ++++ | ++++ |
| 11 | +++ | ++++ | ++++ |
| 12 | ++ | ++++ | ++++ |
| 13 | + | ++++ | ++++ |
| 14 | +++ | ++++ | ++++ |
| 15 | + | ++++ | ++++ |
| 16 | + | +++++ | +++++ |
| 17 | ++ | ++++ | ++++ |
| 18 | + | ++++ | ++++ |
| 19 | +++ | ++++ | ++++ |
| 20 | + | ++++ | +++++ |
| 21 | + | ++++ | ++++ |
| 22 | +++ | ++++ | ++++ |
| 23 | ++ | ++++ | ++++ |
| 24 | + | ++++ | ++++ |
| 25 | +++ | ++++ | |
| 26 | + | +++++ | +++++ |
| 27 | + | ++++ | ++++ |
| 28 | ++ | ++++ | ++++ |
| 29 | +++ | ++++ | ++++ |
| 30 | + | +++++ | +++++ |
| 31 | +++ | ++++ | ++++ |
| 32 | + | +++++ | ++++ |
| 33 | +++ | +++++ | ++++ |
| 34 | ++ | +++++ | +++++ |
| 35 | +++ | ++++ | ++++ |
| 36 | + | +++++ | ++++ |
| 37 | + | ++++ | +++++ |
| 38 | ++ | ++++ | ++++ |
| 39 | ++ | +++++ | ++++ |
| 40 | +++ | +++++ | ++++ |
| 41 | ++ | ++++ | ++++ |
| 42 | + | ++++ | ++++ |
| 43 | +++ | +++++ | ++++ |
| 44 | ++ | ++++ | +++++ |
| 45 | +++ | +++++ | ++++ |
| 46 | + | ++++ | ++++ |
| 47 | + | ++++ | ++++ |
| 48 | +++ | ++++ | ++++ |
| 49 | + | ++++ | +++++ |
| 50 | ++ | ++++ | ++++ |
| 51 | + | +++++ | +++++ |
| 52 | +++ | +++++ | ++++ |
| 53 | + | +++++ | ++++ |
| 54 | ++ | ++++ | ++++ |

**Table 5: Catalysis of Substrate 3**

| Enzyme No. | Wild-type activity | Mutant activity (W60A) | Mutant activity (C61A) |
|---|---|---|---|
| CvTA | ++ | ++++ | ++++ |
| 1 | ++ | +++ | +++++ |
| 2 | +++ | ++++ | +++++ |
| 3 | + | +++ | +++ |
| 4 | +++ | ++++ | ++++ |
| 5 | +++ | ++++ | +++ |
| 6 | ++ | ++++ | ++++ |
| 7 | + | +++++ | ++++ |
| 8 | +++ | ++++ | ++++ |
| 9 | + | ++++ | ++++ |
| 10 | + | +++++ | ++++ |
| 11 | +++ | +++ | +++ |
| 12 | ++ | ++++ | ++++ |
| 13 | + | ++++ | +++ |
| 14 | +++ | ++++ | ++++ |
| 15 | + | ++++ | ++++ |
| 16 | + | +++++ | +++++ |
| 17 | ++ | ++++ | ++++ |
| 18 | + | ++++ | ++++ |
| 19 | +++ | +++ | ++++ |
| 20 | + | ++++ | +++ |
| 21 | + | +++++ | +++ |
| 22 | +++ | ++++ | ++++ |
| 23 | ++ | ++ | ++ |
| 24 | + | ++++ | ++++ |
| 25 | +++ | ++++ | +++ |
| 26 | + | +++++ | ++++ |
| 27 | + | ++++ | ++++ |
| 28 | ++ | ++++ | ++++ |
| 29 | +++ | ++++ | ++++ |
| 30 | + | +++++ | +++++ |
| 31 | +++ | ++++ | ++++ |
| 32 | + | +++++ | +++ |
| 33 | +++ | +++++ | ++++ |
| 34 | ++ | ++++ | +++++ |
| 35 | +++ | ++++ | ++++ |
| 36 | + | +++++ | ++++ |
| 37 | + | ++++ | +++++ |
| 38 | ++ | ++++ | ++++ |
| 39 | ++ | +++++ | ++++ |
| 40 | +++ | +++++ | ++++ |
| 41 | ++ | ++++ | ++++ |
| 42 | + | ++++ | +++ |
| 43 | +++ | +++++ | +++ |
| 44 | ++ | +++ | +++++ |
| 45 | +++ | +++++ | ++++ |
| 46 | + | ++++ | ++++ |
| 47 | + | ++++ | ++++ |
| 48 | +++ | ++ | ++++ |
| 49 | + | ++ | +++ |
| 50 | ++ | +++ | ++++ |
| 51 | + | ++++ | +++++ |
| 52 | +++ | ++++ | +++ |
| 53 | + | ++++ | ++++ |
| 54 | ++ | ++++ | ++++ |

**Table 6: Catalysis of Substrate 4**

| Enzyme No. | Wild-type activity | Mutant activity (W60A) | Mutant activity (C61A) |
|---|---|---|---|
| CvTA | ++ | +++++ | ++++ |
| 1 | ++ | +++ | ++++ |
| 2 | +++ | +++++ | +++++ |
| 3 | + | ++++ | ++++ |
| 4 | +++ | +++ | +++ |
| 5 | +++ | ++++ | ++++ |
| 6 | ++ | +++++ | ++++ |
| 7 | + | +++ | +++ |
| 8 | +++ | +++++ | ++++ |
| 9 | + | ++++ | +++++ |
| 10 | + | +++++ | ++++ |
| 11 | +++ | ++++ | ++++ |
| 12 | ++ | ++++ | ++++ |
| 13 | + | +++ | ++ |
| 14 | +++ | ++++ | ++++ |
| 15 | + | ++++ | ++++ |
| 16 | + | +++ | ++++ |
| 17 | ++ | ++++ | ++++ |
| 18 | + | ++++ | ++++ |
| 19 | +++ | ++++ | +++ |
| 20 | + | ++++ | ++++ |
| 21 | + | +++++ | ++++ |
| 22 | +++ | ++++ | ++++ |
| 23 | ++ | +++ | ++++ |
| 24 | + | ++++ | +++ |
| 25 | +++ | ++++ | +++ |
| 26 | + | +++++ | +++++ |
| 27 | + | ++++ | ++++ |
| 28 | ++ | ++++ | ++++ |
| 29 | +++ | ++++ | ++++ |
| 30 | + | +++++ | +++++ |
| 31 | +++ | ++++ | ++++ |
| 32 | + | +++++ | ++++ |
| 33 | +++ | +++++ | ++++ |
| 34 | ++ | +++++ | +++++ |
| 35 | +++ | ++++ | ++++ |
| 36 | + | +++++ | ++++ |
| 37 | + | ++++ | +++++ |
| 38 | ++ | ++++ | ++++ |
| 39 | ++ | +++++ | ++++ |
| 40 | +++ | +++++ | +++ |
| 41 | ++ | ++++ | ++++ |
| 42 | + | ++ | ++++ |
| 43 | +++ | +++++ | ++++ |
| 44 | ++ | ++++ | +++ |
| 45 | +++ | +++++ | ++++ |
| 46 | + | ++++ | ++++ |
| 47 | + | ++++ | ++++ |
| 48 | +++ | ++++ | ++++ |
| 49 | + | ++++ | ++++ |
| 50 | ++ | ++++ | ++++ |
| 51 | + | +++++ | +++++ |
| 52 | +++ | ++++ | ++++ |
| 53 | + | +++++ | +++ |
| 54 | ++ | ++++ | ++++ |

**Table 7: Catalysis of Substrate 5**

| Enzyme No. | Wild-type activity | Mutant activity (W60A) | Mutant activity (C61A) |
|---|---|---|---|
| CvTA | ++ | +++++ | +++ |
| 1 | ++ | +++ | +++++ |
| 2 | +++ | +++++ | +++++ |
| 3 | + | ++++ | +++ |
| 4 | +++ | +++ | ++++ |
| 5 | +++ | ++++ | +++ |
| 6 | ++ | +++++ | ++++ |
| 7 | + | ++++ | ++++ |
| 8 | +++ | +++++ | ++++ |
| 9 | + | ++++ | +++++ |
| 10 | + | +++++ | ++++ |
| 11 | +++ | ++++ | +++ |
| 12 | ++ | ++++ | ++++ |
| 13 | + | +++++ | ++++ |
| 14 | +++ | ++++ | ++++ |
| 15 | + | ++++ | ++++ |
| 16 | + | ++++ | +++++ |
| 17 | ++ | ++++ | +++ |
| 18 | + | ++++ | +++ |
| 19 | +++ | ++++ | ++++ |
| 20 | + | ++++ | +++++ |
| 21 | + | +++++ | ++++ |
| 22 | +++ | ++++ | +++ |
| 23 | ++ | ++++ | ++++ |
| 24 | + | ++++ | ++++ |
| 25 | +++ | ++++ | +++ |
| 26 | + | +++++ | ++++ |
| 27 | + | ++++ | ++++ |
| 28 | ++ | ++++ | ++++ |
| 29 | +++ | ++++ | +++ |
| 30 | + | +++++ | +++ |
| 31 | +++ | +++ | +++ |
| 32 | + | ++++ | ++++ |
| 33 | +++ | +++++ | ++++ |
| 34 | ++ | +++++ | +++ |
| 35 | +++ | +++ | +++ |
| 36 | + | +++++ | ++++ |
| 37 | + | ++++ | +++++ |
| 38 | ++ | ++++ | ++++ |
| 39 | ++ | ++++ | +++ |
| 40 | +++ | +++++ | ++++ |
| 41 | ++ | +++ | +++ |
| 42 | + | ++++ | +++ |
| 43 | +++ | +++++ | ++++ |
| 44 | ++ | ++++ | +++ |
| 45 | +++ | +++++ | ++++ |
| 46 | + | ++++ | ++++ |
| 47 | + | ++ | ++++ |
| 48 | +++ | ++++ | ++ |
| 49 | + | ++++ | +++++ |
| 50 | ++ | ++++ | ++++ |
| 51 | + | +++ | +++ |
| 52 | +++ | ++++ | ++++ |
| 53 | + | +++ | +++ |
| 54 | ++ | ++++ | ++++ |

**Table 8: Catalysis of Substrate 6**

| Enzyme No. | Wild-type activity | Mutant activity (W60A) | Mutant activity (C61A) |
|---|---|---|---|
| CvTA | ++ | +++++ | ++++ |
| 1 | ++ | ++++ | ++++ |
| 2 | +++ | +++++ | +++++ |
| 3 | + | ++++ | +++ |
| 4 | +++ | ++++ | ++++ |
| 5 | +++ | +++ | ++++ |
| 6 | ++ | +++++ | ++++ |
| 7 | + | ++++ | ++++ |
| 8 | +++ | ++++ | ++++ |
| 9 | + | ++++ | ++++ |
| 10 | + | ++++ | ++++ |
| 11 | +++ | ++++ | ++++ |
| 12 | ++ | ++++ | ++++ |
| 13 | + | iiiii | +++ |
| 14 | +++ | +++ | ++++ |
| 15 | + | ++++ | ++++ |
| 16 | + | ++++ | ++++ |
| 17 | ++ | ++++ | ++++ |
| 18 | + | ++++ | ++++ |
| 19 | +++ | +++ | +++ |
| 20 | + | ++++ | ++++ |
| 21 | + | +++ | ++++ |
| 22 | +++ | +++ | ++++ |
| 23 | ++ | ++++ | +++ |
| 24 | + | +++ | ++++ |
| 25 | +++ | ++++ | ++++ |
| 26 | + | +++++ | ++++ |
| 27 | + | ++++ | ++++ |
| 28 | ++ | ++++ | ++++ |
| 29 | +++ | +++ | ++++ |
| 30 | + | +++++ | ++++ |
| 31 | +++ | ++++ | ++++ |
| 32 | + | +++++ | +++ |
| 33 | +++ | ++++ | ++++ |
| 34 | ++ | +++++ | +++++ |
| 35 | +++ | +++ | ++++ |
| 36 | + | +++++ | +++ |
| 37 | + | ++++ | ++++ |
| 38 | ++ | ++++ | ++++ |
| 39 | ++ | +++++ | ++++ |
| 40 | +++ | ++++ | ++++ |
| 41 | ++ | ++++ | ++++ |
| 42 | + | ++++ | ++++ |
| 43 | +++ | +++++ | ++++ |
| 44 | ++ | ++++ | +++++ |
| 45 | +++ | +++++ | +++ |
| 46 | + | ++++ | ++++ |
| 47 | + | +++ | ++++ |
| 48 | +++ | ++++ | ++++ |
| 49 | + | ++++ | +++++ |
| 50 | ++ | ++++ | +++ |
| 51 | + | +++++ | ++++ |
| 52 | +++ | ++++ | ++++ |
| 53 | + | +++++ | ++++ |
| 54 | ++ | ++++ | ++++ |

**Table 9: Catalysis of Substrate 7**

| Enzyme No. | Wild-type activity | Mutant activity (W60A) | Mutant activity (C61A) |
|---|---|---|---|
| CvTA | ++ | +++++ | ++++ |
| 1 | ++ | ++++ | +++++ |
| 2 | +++ | +++++ | +++++ |
| 3 | + | ++ | ++++ |
| 4 | +++ | ++++ | ++++ |
| 5 | +++ | ++++ | ++++ |
| 6 | ++ | +++++ | ++++ |
| 7 | + | +++++ | ++ |
| 8 | +++ | +++++ | ++++ |
| 9 | + | ++++ | +++++ |
| 10 | + | +++++ | +++ |
| 11 | +++ | ++++ | ++++ |
| 12 | ++ | ++++ | ++++ |
| 13 | + | +++++ | ++++ |
| 14 | +++ | ++++ | ++++ |
| 15 | + | ++++ | ++++ |
| 16 | + | +++++ | +++++ |
| 17 | ++ | ++++ | ++++ |
| 18 | + | ++++ | ++++ |
| 19 | +++ | ++++ | ++++ |
| 20 | + | ++++ | +++++ |
| 21 | + | ++++ | ++++ |
| 22 | +++ | ++++ | ++++ |
| 23 | ++ | ++++ | ++++ |
| 24 | + | ++++ | ++++ |
| 25 | +++ | ++++ | +++++ |
| 26 | + | +++++ | +++++ |
| 27 | + | ++++ | ++++ |
| 28 | ++ | ++++ | ++++ |
| 29 | +++ | ++++ | ++++ |
| 30 | + | ++++ | +++++ |
| 31 | +++ | ++++ | ++++ |
| 32 | + | +++++ | ++++ |
| 33 | +++ | +++++ | +++ |
| 34 | ++ | +++++ | +++++ |
| 35 | +++ | ++++ | ++++ |
| 36 | + | ++++ | ++++ |
| 37 | + | ++++ | ++++ |
| 38 | ++ | ++++ | ++++ |
| 39 | ++ | +++++ | ++++ |
| 40 | +++ | +++++ | ++++ |
| 41 | ++ | ++++ | ++++ |
| 42 | + | ++++ | ++++ |
| 43 | +++ | +++++ | ++++ |
| 44 | ++ | ++++ | ++++ |
| 45 | +++ | +++++ | ++++ |
| 46 | + | +++ | ++++ |
| 47 | + | ++++ | ++++ |
| 48 | +++ | ++++ | ++++ |
| 49 | + | ++++ | +++++ |
| 50 | ++ | ++++ | ++++ |
| 51 | + | +++++ | +++++ |
| 52 | +++ | +++++ | ++++ |
| 53 | + | +++++ | ++++ |
| 54 | ++ | ++++ | ++++ |

**Table 10: Catalysis of Substrate 8**

| Enzyme No. | Wild-type activity | Mutant activity (W60A) | Mutant activity (C61A) |
|---|---|---|---|
| CvTA | ++ | ++++ | +++ |
| 1 | ++ | ++++ | +++++ |
| 2 | +++ | ++++ | ++++ |
| 3 | + | ++++ | ++++ |
| 4 | +++ | ++++ | ++++ |
| 5 | +++ | ++++ | ++++ |
| 6 | ++ | +++++ | ++++ |
| 7 | + | +++++ | ++++ |
| 8 | +++ | +++++ | ++++ |
| 9 | + | ++++ | ++++ |
| 10 | + | +++++ | ++++ |
| 11 | +++ | ++++ | ++++ |
| 12 | ++ | ++++ | ++++ |
| 13 | + | +++++ | ++++ |
| 14 | +++ | ++++ | ++++ |
| 15 | + | ++++ | ++++ |
| 16 | + | +++++ | +++++ |
| 17 | ++ | ++++ | ++++ |
| 18 | + | ++++ | ++++ |
| 19 | +++ | ++++ | ++++ |
| 20 | + | ++++ | +++ |
| 21 | + | +++++ | ++++ |
| 22 | +++ | ++++ | ++++ |
| 23 | ++ | ++++ | ++++ |
| 24 | + | ++++ | ++++ |
| 25 | +++ | ++++ | +++++ |
| 26 | + | +++++ | +++++ |
| 27 | + | ++++ | ++++ |
| 28 | ++ | ++++ | ++++ |
| 29 | +++ | ++++ | ++++ |
| 30 | + | +++ | +++++ |
| 31 | +++ | ++++ | ++++ |
| 32 | + | +++++ | ++++ |
| 33 | +++ | +++++ | ++++ |
| 34 | ++ | +++++ | +++ |
| 35 | +++ | ++++ | ++++ |
| 36 | + | +++++ | ++++ |
| 37 | + | ++++ | +++++ |
| 38 | ++ | ++++ | ++++ |
| 39 | ++ | +++++ | ++++ |
| 40 | +++ | +++++ | ++++ |
| 41 | ++ | ++++ | ++++ |
| 42 | + | ++++ | ++++ |
| 43 | +++ | +++++ | ++++ |
| 44 | ++ | ++++ | +++++ |
| 45 | +++ | +++++ | ++++ |
| 46 | + | +++ | ++++ |
| 47 | + | ++++ | ++++ |
| 48 | +++ | ++++ | ++++ |
| 49 | + | ++++ | +++++ |
| 50 | ++ | ++++ | ++++ |
| 51 | + | +++++ | +++++ |
| 52 | +++ | +++++ | ++ |
| 53 | + | +++++ | ++++ |
| 54 | ++ | ++++ | ++++ |

**Table 11: Catalysis of Substrate 9**

| Enzyme No. | Wild-type activity | Mutant activity (W60A) | Mutant activity (C61A) |
|---|---|---|---|
| CvTA | ++ | +++++ | ++++ |
| 1 | ++ | ++++ | +++++ |
| 2 | +++ | ++++ | +++ |
| 3 | + | ++++ | ++++ |
| 4 | +++ | ++++ | ++++ |
| 5 | +++ | ++++ | ++++ |
| 6 | ++ | +++++ | ++++ |
| 7 | + | +++++ | ++++ |
| 8 | +++ | +++++ | ++++ |
| 9 | + | ++++ | +++++ |
| 10 | + | +++++ | ++++ |
| 11 | +++ | ++++ | ++ |
| 12 | ++ | ++++ | ++++ |
| 13 | + | +++++ | ++++ |
| 14 | +++ | ++++ | ++++ |
| 15 | + | ++++ | ++++ |
| 16 | + | +++++ | +++++ |
| 17 | ++ | ++++ | ++++ |
| 18 | + | ++++ | ++++ |
| 19 | +++ | ++ | ++++ |
| 20 | + | ++++ | +++++ |
| 21 | + | ++++ | ++++ |
| 22 | +++ | ++++ | ++++ |
| 23 | ++ | ++++ | ++++ |
| 24 | + | ++++ | ++++ |
| 25 | +++ | ++++ | +++ |
| 26 | + | +++++ | +++++ |
| 27 | + | ++++ | ++++ |
| 28 | ++ | +++ | ++++ |
| 29 | +++ | ++++ | ++++ |
| 30 | + | +++++ | +++++ |
| 31 | +++ | ++++ | ++++ |
| 32 | + | +++++ | ++++ |
| 33 | +++ | +++++ | ++++ |
| 34 | ++ | +++++ | +++++ |
| 35 | +++ | ++++ | +++ |
| 36 | + | +++++ | ++++ |
| 37 | + | ++++ | +++++ |
| 38 | ++ | ++++ | ++++ |
| 39 | ++ | +++++ | ++++ |
| 40 | +++ | +++++ | ++++ |
| 41 | ++ | ++++ | ++++ |
| 42 | + | ++++ | ++++ |
| 43 | +++ | +++ | ++++ |
| 44 | ++ | ++++ | +++ |
| 45 | +++ | +++++ | ++++ |
| 46 | + | ++++ | ++++ |
| 47 | + | ++++ | ++++ |
| 48 | +++ | ++++ | ++++ |
| 49 | + | ++++ | +++++ |
| 50 | ++ | ++++ | ++++ |
| 51 | + | +++++ | +++ |
| 52 | +++ | +++ | ++++ |
| 53 | + | +++++ | ++++ |
| 54 | ++ | ++++ | ++++ |

**Table 12: Catalysis of Substrate 10**

| Enzyme No. | Wild-type activity | Mutant activity (W60A) | Mutant activity (C61A) |
|---|---|---|---|
| CvTA | ++ | +++++ | +++ |
| 1 | ++ | ++++ | +++++ |
| 2 | +++ | +++ | ++++ |
| 3 | + | ++++ | ++++ |
| 4 | +++ | ++++ | ++++ |
| 5 | +++ | ++++ | ++++ |
| 6 | ++ | +++++ | ++++ |
| 7 | + | +++++ | ++++ |
| 8 | +++ | +++ | ++++ |
| 9 | + | +++ | ++++ |
| 10 | + | +++++ | ++++ |
| 11 | +++ | ++++ | ++++ |
| 12 | ++ | ++++ | ++++ |
| 13 | + | +++++ | ++++ |
| 14 | +++ | ++++ | ++++ |
| 15 | + | ++++ | ++++ |
| 16 | + | +++ | ++++ |
| 17 | ++ | ++++ | +++ |
| 18 | + | ++++ | ++++ |
| 19 | +++ | ++++ | ++++ |
| 20 | + | ++++ | ++++ |
| 21 | + | +++++ | ++++ |
| 22 | +++ | ++++ | ++++ |
| 23 | ++ | ++++ | ++++ |
| 24 | + | ++++ | ++++ |
| 25 | +++ | ++++ | +++ |
| 26 | + | +++++ | +++++ |
| 27 | + | ++++ | ++++ |
| 28 | ++ | ++++ | ++++ |
| 29 | +++ | ++++ | ++++ |
| 30 | + | +++++ | +++++ |
| 31 | +++ | ++++ | ++++ |
| 32 | + | +++++ | ++++ |
| 33 | +++ | +++++ | ++++ |
| 34 | ++ | +++++ | +++++ |
| 35 | +++ | ++++ | ++++ |
| 36 | + | +++++ | ++++ |
| 37 | + | ++++ | +++++ |
| 38 | ++ | ++++ | ++++ |
| 39 | ++ | +++++ | ++++ |
| 40 | +++ | +++++ | ++++ |
| 41 | ++ | ++++ | ++++ |
| 42 | + | ++++ | ++++ |
| 43 | +++ | +++++ | ++++ |
| 44 | ++ | ++++ | +++++ |
| 45 | +++ | +++++ | ++++ |
| 46 | + | ++++ | ++++ |
| 47 | + | +++ | ++++ |
| 48 | +++ | ++++ | ++++ |
| 49 | + | ++++ | ++++ |
| 50 | ++ | ++++ | ++++ |
| 51 | + | +++++ | +++ |
| 52 | +++ | +++ | ++++ |
| 53 | + | +++++ | ++++ |
| 54 | ++ | ++++ | ++++ |

**Table 13: Catalysis of Substrate 11**

| Enzyme No. | Wild-type activity | Mutant activity (W60A) | Mutant activity (C61A) |
|---|---|---|---|
| CvTA | ++ | +++++ | ++++ |
| 1 | ++ | ++++ | +++++ |
| 2 | +++ | +++++ | +++++ |
| 3 | + | +++ | ++++ |
| 4 | +++ | ++++ | ++++ |
| 5 | +++ | ++++ | ++++ |
| 6 | ++ | +++++ | ++++ |
| 7 | + | +++++ | ++++ |
| 8 | +++ | +++++ | ++++ |
| 9 | + | ++++ | +++++ |
| 10 | + | +++++ | ++++ |
| 11 | +++ | ++++ | ++++ |
| 12 | ++ | ++++ | ++++ |
| 13 | + | +++++ | ++++ |
| 14 | +++ | ++++ | ++++ |
| 15 | + | ++++ | ++++ |
| 16 | + | +++++ | +++ |
| 17 | ++ | +++ | ++++ |
| 18 | + | ++++ | ++++ |
| 19 | +++ | ++++ | ++++ |
| 20 | + | ++++ | +++++ |
| 21 | + | +++++ | +++ |
| 22 | +++ | ++++ | ++++ |
| 23 | ++ | ++++ | ++++ |
| 24 | + | ++++ | ++++ |
| 25 | +++ | ++++ | +++ |
| 26 | + | +++++ | +++++ |
| 27 | + | ++++ | ++++ |
| 28 | ++ | ++++ | ++++ |
| 29 | +++ | ++++ | ++++ |
| 30 | + | +++++ | +++++ |
| 31 | +++ | ++++ | ++++ |
| 32 | + | +++++ | ++++ |
| 33 | +++ | +++++ | ++++ |
| 34 | ++ | +++++ | +++++ |
| 35 | +++ | ++++ | ++++ |
| 36 | + | +++ | ++++ |
| 37 | + | ++++ | +++++ |
| 38 | ++ | ++++ | ++++ |
| 39 | ++ | +++++ | ++++ |
| 40 | +++ | +++++ | ++++ |
| 41 | ++ | ++++ | ++++ |
| 42 | + | ++++ | ++ |
| 43 | +++ | +++++ | ++++ |
| 44 | ++ | ++++ | +++++ |
| 45 | +++ | +++++ | ++++ |
| 46 | + | ++++ | ++++ |
| 47 | + | ++++ | ++++ |
| 48 | +++ | ++++ | ++ |
| 49 | + | ++ | +++++ |
| 50 | ++ | ++++ | ++++ |
| 51 | + | +++++ | +++++ |
| 52 | +++ | +++++ | ++++ |
| 53 | + | +++++ | ++++ |
| 54 | ++ | ++++ | ++++ |

**Table 14: Catalysis of Substrate 12**

| Enzyme No. | Wild-type activity | Mutant activity (W60A) | Mutant activity (C61A) |
|---|---|---|---|
| CvTA | ++ | +++++ | ++++ |
| 1 | ++ | ++++ | +++++ |
| 2 | +++ | +++++ | +++++ |
| 3 | + | ++++ | ++++ |
| 4 | +++ | ++ | ++++ |
| 5 | +++ | ++++ | ++++ |
| 6 | ++ | +++++ | ++++ |
| 7 | + | +++++ | ++++ |
| 8 | +++ | +++++ | ++++ |
| 9 | + | ++++ | +++++ |
| 10 | + | +++++ | ++++ |
| 11 | +++ | ++++ | ++++ |
| 12 | ++ | ++++ | ++++ |
| 13 | + | +++++ | ++++ |
| 14 | +++ | ++++ | ++++ |
| 15 | + | ++++ | ++ |
| 16 | + | +++++ | +++++ |
| 17 | ++ | ++++ | ++++ |
| 18 | + | ++++ | ++++ |
| 19 | +++ | ++++ | ++++ |
| 20 | + | ++ | ++++ |
| 21 | + | +++++ | ++++ |
| 22 | +++ | ++++ | ++++ |
| 23 | ++ | ++++ | ++++ |
| 24 | + | ++++ | ++++ |
| 25 | +++ | ++++ | +++ |
| 26 | + | +++++ | +++++ |
| 27 | + | ++++ | ++++ |
| 28 | ++ | ++++ | ++++ |
| 29 | +++ | +++ | ++++ |
| 30 | + | +++++ | +++++ |
| 31 | +++ | +++ | ++++ |
| 32 | + | +++++ | ++++ |
| 33 | +++ | +++++ | ++++ |
| 34 | ++ | +++++ | +++++ |
| 35 | +++ | ++++ | ++++ |
| 36 | + | +++++ | ++++ |
| 37 | + | ++++ | +++++ |
| 38 | ++ | ++++ | ++++ |
| 39 | ++ | +++++ | +++ |
| 40 | +++ | +++++ | ++++ |
| 41 | ++ | +++ | ++++ |
| 42 | + | ++++ | ++++ |
| 43 | +++ | +++++ | ++++ |
| 44 | ++ | ++++ | +++++ |
| 45 | +++ | +++++ | ++++ |
| 46 | + | ++++ | +++ |
| 47 | + | ++++ | ++++ |
| 48 | +++ | ++++ | ++++ |
| 49 | + | ++++ | +++++ |
| 50 | ++ | ++++ | ++++ |
| 51 | + | +++++ | +++++ |
| 52 | +++ | +++ | ++++ |
| 53 | + | +++++ | +++ |
| 54 | ++ | ++++ | ++++ |

**Table 15: Catalysis of Substrate 13**

| Enzyme No. | Wild-type activity | Mutant activity (W60A) | Mutant activity (C61A) |
|---|---|---|---|
| CvTA | +++ | ++++ | ++++ |
| 1 | +++ | +++++ | ++++ |
| 2 | +++ | +++++ | +++++ |
| 3 | + | ++++ | ++++ |
| 4 | +++ | ++++ | +++++ |
| 5 | +++ | ++++ | ++++ |
| 6 | ++ | ++++ | ++++ |
| 7 | ++ | +++ | ++++ |
| 8 | +++ | +++++ | ++++ |
| 9 | + | ++++ | +++++ |
| 10 | ++ | +++++ | ++++ |
| 11 | +++ | ++++ | ++++ |
| 12 | ++ | ++++ | ++++ |
| 13 | ++ | +++ | +++++ |
| 14 | +++ | ++++ | ++++ |
| 15 | + | ++++ | ++++ |
| 16 | ++ | +++ | ++++ |
| 17 | ++ | ++++ | ++++ |
| 18 | + | ++++ | ++++ |
| 19 | +++ | ++++ | +++++ |
| 20 | ++ | ++++ | ++++ |
| 21 | ++ | +++++ | ++++ |
| 22 | +++ | ++++ | ++++ |
| 23 | ++ | +++ | ++++ |
| 24 | + | ++++ | +++ |
| 25 | +++ | ++++ | +++ |
| 26 | + | +++++ | +++++ |
| 27 | ++ | ++++ | ++++ |
| 28 | ++ | ++++ | ++++ |
| 29 | +++ | ++++ | ++++ |
| 30 | + | +++++ | +++++ |
| 31 | +++ | ++++ | ++++ |
| 32 | ++ | +++++ | ++++ |
| 33 | +++ | +++++ | ++++ |
| 34 | ++ | +++++ | +++++ |
| 35 | +++ | ++++ | ++++ |
| 36 | ++ | +++++ | ++++ |
| 37 | + | ++++ | +++++ |
| 38 | ++ | ++++ | ++++ |
| 39 | ++ | +++++ | ++++ |
| 40 | +++ | +++++ | +++ |
| 41 | ++ | ++++ | ++++ |
| 42 | + | ++ | ++++ |
| 43 | +++ | +++++ | ++++ |
| 44 | ++ | ++++ | +++ |
| 45 | +++ | +++++ | ++++ |
| 46 | + | ++++ | ++++ |
| 47 | + | ++++ | ++++ |
| 48 | +++ | ++++ | ++++ |
| 49 | + | ++++ | ++++ |
| 50 | ++ | ++++ | ++++ |
| 51 | + | +++++ | +++++ |
| 52 | +++ | ++++ | ++++ |
| 53 | ++ | +++++ | +++ |
| 54 | ++ | ++++ | ++++ |

From the above description, it may be seen that the above embodiments of the present disclosure achieve the following technical effects: 1) the above wild-type transaminases with the conserved regions from various sources are used for biocatalysis, and the noble metal catalyst does not need to be used, to achieve the green chemistry; 2) compared with the noble metal catalyst, the transaminase catalytic reaction adopted is short in synthesizing route, it is helpful to reduce the three wastes and save the production cost; 3) the transaminases from different sources are used to obtain the transaminase mutants after the conserved site mutation, the biotransformation reaction of catalyzing the large sterically hindered ketone substrate may be improved; and 4) the substrate types are widely used.

The above are only preferred embodiments of the present disclosure and are not intended to limit the present disclosure. For those skilled in the art, the present disclosure may have various variations and changes. Any modifications, equivalent replacements, improvements and the like made within the spirit and principles of the present disclosure shall be included within the scope of protection of the present disclosure.

## Claims

1. A method for synthesizing a chiral amine compound, comprising:
performing a transamination reaction on a ketone substrate of formula I with a transaminase under the action of an amino donor;
obtaining the chiral amine compound; 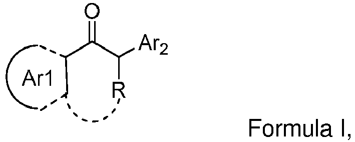
wherein Ar1 represents a substituted or unsubstituted aryl, a substituted or unsubstituted arylene, or a substituted or unsubstituted heteroarylene;
Ar2 represents a substituted or unsubstituted aryl, a substituted or unsubstituted cycloalkyl, or a substituted or unsubstituted chain alkyl, or a substituted or unsubstituted heteroalkyl; and
optionally R represents a substituted or unsubstituted C1-C5 alkylene, and the R is connected to Ar1 to form a ring;
wherein the substituents in the substituted aryl, the substituted arylene, the substituted heteroarylene and substituted alkylene are each independently selected from a halogen, a hydroxy, an amino group, or -S-CH₃, and the heteroatoms in the heteroarylene and the heteroalkyl are each independently selected from N, O or S;
wherein the transaminase is a type of transaminases with a conserved amino acid sequence region, and the conserved amino acid sequence region comprises at least a region 1 and a region 2, the conserved amino acid sequence of the region 1 is: MAGLWCVN, and the conserved amino acid sequence of the region 2 is: YNTFFKT;
the transaminase is *Chromobaterium violaceum* shown in SEQ ID NO: 1 and a mutated transaminase of any one transaminases shown in SEQ ID NO: 2 to SEQ ID NO: 55 of which W and/or C in the conserved amino acid sequence of the region 1 is mutated into A; or the transaminase is a transaminase which has the same source as any one of the transaminases shown in SEQ ID NO: 1 to SEQ ID NO: 55 and has more than 95% of identity on the premise of having the region 1 and the region 2:
| No. | Source | NCBI Seq ID | SEQ ID NO: |
|---|---|---|---|
| 1 | Chromobacterium vaccinii | WP_046156378.1 | 2 |
| 2 | Chromobacterium subtsugae | WP_047243213.1 | 3 |
| 3 | Chromobacterium sphagni | WP_0711168S6.1 | a |
| a | unclassified Chromobacterium | WP_114062556.1 | 5 |
| 5 | Chromobacterium vaccinii | WP_104946997.1 | 6 |
| 6 | Chromobacterium sp. 257-1 | WP_14939S777.1 | 7 |
| 7 | Chromobacterium sp. MWU14-2602 | WP_103903523.1 | 8 |
| a | Chromobacterium sp. ATCC 53434 | WP_101708025.1 | 9 |
| 9 | Chromobacterium | WP_043629242.1 | 10 |
| 10 | Xenophilus sp. AP218F | WP_088737038.1 | 11 |
| 11 | Chromobacterium sp. LK11 | WP_048412320.1 | 12 |
| | | | |
| 12 | Chromobacterium sp. LK1 | WP_048411976.1 | 13 |
| 13 | Chromobacterium haemolyticum | WP_081556739.1 | 14 |
| 14 | Chromobacterium haemolyticum | OQS33371 1 | 15 |
| 15 | Chromobactenum sp. Panama | WP_107199474.1 | 16 |
| 16 | Chromobacterium haemolyticum | OQS37730.1 | 17 |
| 17 | Chromobacterium haemolyticum | WP_166453011.1 | 18 |
| 18 | Vogesellaoryzae | WP_174874069.1 | 19 |
| 19 | Vogesellaurethralis | WP_144311115.1 | 20 |
| 20 | Vogesellasp.LIG4 | WP_088967522.1 | 21 |
| 21 | Aquitalea | WP_089085350.1 | 22 |
| 22 | Aquitaleasp.LB_tupeE | WP_178913910.1 | 23 |
| 23 | Vogesellaperiucida | WP_147687830.1 | 24 |
| 24 | Aquitalea | WP_103523625.1 | 15 |
| 25 | Aquitaleadenitrificans | WP_159877955.1 | 16 |
| 26 | Aquitalea | WP_045848621.1 | 27 |
| 21 | Aquttaleasp.FJL05 | WP_124643387.1 | 28 |
| 28 | Aquitaleamagnusonii | WP_059297319.1 | 29 |
| 29 | Pseudogulbenkianiasp.MAI-1 | WP_024302818.1 | 30 |
| 30 | Paludibacteriumpaludis | WP_189532963.1 | 31 |
| 31 | Pseudogulbenkianiasp.NH8B | WP_014087389.1 | 32 |
| 32 | Pseudogulbeukianiasubflava | WP_085275708.1 | 33 |
| 33 | Pseudogulbenkianiaferrooxidans | WP_008952788.1 | 34 |
| 34 | Gulbenkianiaindica | WP_055434103.1 | 35 |
| 35 | Paludibacteriumsp.dN18-1 | MTD33855.1 | 36 |
| 36 | Vogtsellafluminis | WP_189352298.1 | 37 |
| 37 | Vogesellaalkaliphila | WP_189374996.1 | 38 |
| 38 | Crenobactersp.HX-7-9 | WP_163315775.1 | 39 |
| 39 | Vogesellaindigofera | WP_120809711.1 | 40 |
| 40 | Crenobactersp.GY70310 | WP_136552942.1 | 41 |
| 41 | Vogesellasp.EB | WP_047966302.1 | 42 |
| 42 | RhodobacteraceaebacteriumCH30 | RQW28969.1 | 43 |
| 43 | NeisseriaceaebacteriumB2N2-7 | MXR37125.1 | 44 |
| 44 | Microvirgula | WP_028499438.1 | 45 |
| 45 | Neisseriashayeganii | WP_182122880.1 | 46 |
| 46 | Netsseriashayeganii | WP_009118141.1 | 47 |
| 47 | Vitreoscillasp.C1 | AAD41041.1 | 48 |
| 48 | Vitreoscilla | WP_019957606.1 | 49 |
| 49 | Lanbacterbongkongensis | WP_041825665.1 | 50 |
| 50 | Laribacterhongkongensis | WP_1935844351 | 51 |
| 51 | Rivicolapingtungensis | WP_10389486.1 | 52 |
| 52 | Srenoxybacteracetivorans | WP_037587322.1 | 53 |
| 33 | Aquaspririllumsp.LM1 | WP_077297579.1 | 54 |
| 54 | Paludibacteriumyongneupense | WP_028535161.1 | 55 |

2. The method according to claim 1, wherein the transaminase is any one of transaminases derived from *Chromobaterium violaceum* and the species shown in Table 1 and Table 2 with enzyme numbers 1 to 54.

3. The method according to claim 1, wherein the substituents in the substituted aryl and the substituted arylene are each individually selected from a halogen or -S-CH₃, and the halogen or -S-CH₃ is located at any one or more positions of ortho, meta or para position of the aryl or arylene.

4. The method according to claim 1, wherein the Ar1 represents a substituted or unsubstituted aryl, or a substituted or unsubstituted arylene, the Ar2 represents an unsubstituted aryl, and the R represents an unsubstituted C1~C5 alkylene, and the R is connected to Ar1 to form a ring.

5. The method according to claim 1, wherein the Ar1 represents an unsubstituted heteroarylene, the Ar2 represents a halogen-substituted aryl, and the R represents a hydroxylsubstituted C1~C5 alkylene, and the R is connected to Ar1 to form a ring.

6. The method according to claim 1, wherein the Ar1 represents a halogen- and aminosubstituted arylene, and the Ar2 represents a C3∼C8 cycloalkyl.

7. The method according to claim 1, wherein the Ar1 represents an unsubstituted cycloalkyl or an unsubstituted aryl, and the Ar2 represents a substituted or unsubstituted cycloalkyl, or a substituted or unsubstituted aryl.

8. The method according to claim 1, wherein the Ar1 represents a hydroxyl-, methyl-, ethyl-, or -S-CH₃-substituted cycloalkyl, or a hydroxyl-, methyl-, ethyl-, or -S-CH₃-substituted aryl, and the Ar2 represents an unsubstituted aryl or an unsubstituted alkyl.

9. The method according to claim 1, wherein the ketone substrate is selected from the group consisting of:

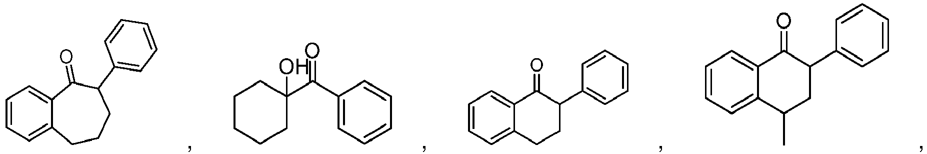

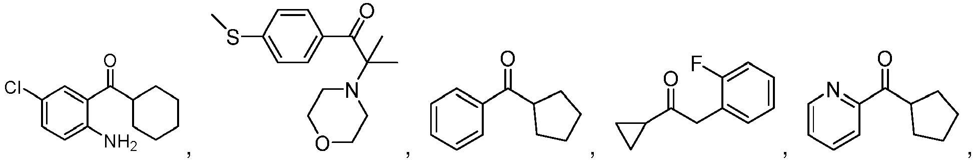

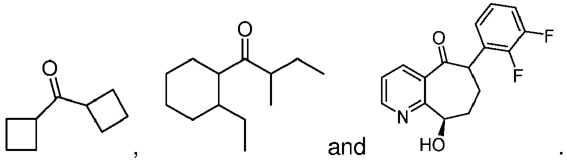

10. The method according to claim 1, wherein the amino donor is selected from the group consisting of isopropylamine, isopropylamine hydrochloride, alanine, n-butylamine and aniline.
